Europäisches Patentamt

European Patent Office

Office européen des brevets

(19)

(11) **EP 0 946 280 B1**

(12) **EUROPÄISCHE PATENTSCHRIFT**

(45) Veröffentlichungstag und Bekanntmachung des Hinweises auf die Patenterteilung:
**28.05.2003 Patentblatt 2003/22**

(51) Int Cl.7: **B01J 19/00**, B01J 10/00, B01J 19/14

(21) Anmeldenummer: **97912145.6**

(22) Anmeldetag: **08.10.1997**

(86) Internationale Anmeldenummer:
**PCT/EP97/05542**

(87) Internationale Veröffentlichungsnummer:
**WO 98/017381 (30.04.1998 Gazette 1998/17)**

(54) **VERFAHREN ZUR DURCHFÜHRUNG VON DURCH EIN GLEICHGEWICHT GEKENNZEICHNETEN REAKTIONEN**

METHOD FOR CARRYING OUT REACTIONS CHARACTERIZED BY AN EQUILIBIRUM

PROCEDE POUR REALISER DES REACTIONS CARACTERISEES PAR UN EQUILIBRE

(84) Benannte Vertragsstaaten:
**BE DE DK FR GB IT NL**

(30) Priorität: **18.10.1996 DE 19643165**

(43) Veröffentlichungstag der Anmeldung:
**06.10.1999 Patentblatt 1999/40**

(73) Patentinhaber: **Ellis, Vincent Simon**
**3078 Everberg (BE)**

(72) Erfinder: **Ellis, Vincent Simon**
**3078 Everberg (BE)**

(74) Vertreter: **Koepe, Gerd L., Dipl.-Chem. et al**
**Koepe & Partner,**
**Robert-Koch-Strasse 1**
**80538 München (DE)**

(56) Entgegenhaltungen:
**EP-A- 0 565 777**     **AT-A- 287 890**
**DE-A- 19 541 213**     **US-A- 5 159 092**

**Beschreibung**

Die vorliegende Erfindung betrifft ein Verfahren zur Durchführung von Reaktionen, die durch ein Gleichgewicht gekennzeichnet sind.

Zahlreiche chemische Reaktionen einschließlich solcher Reaktionen, die in industriellem Maßstab durchgeführt werden, sind dadurch gekennzeichnet, daß sich nach einer gewissen Zeit ein chemisches Gleichgewicht zwischen Edukten und Produkten einstellt, das den weitgehenden Ablauf der chemischen Reaktion unter Bildung der Produkte und damit das Erzielen einer für die wirtschaftliche Durchführung der Reaktion wichtigen hohen Ausbeute der gewünschten Produkte verhindert.

Diesem Problem versucht man dadurch zu begegnen, daß man versucht, das Gleichgewicht einer chemischen Reaktion im gewünschten Sinn zu beeinflussen. Grundlage hierfür bilden das bekannte Massenwirkungsgesetz und die durch dieses definierten, von den Reaktionsbedingungen abhängigen Gleichgewichtskonstanten. Dem Prinzip von Le Chatelier folgend, läuft die Reaktion dann in der gewünschten Weise ab, und es stellt sich ein neues (bei geeigneter Beeinflussung weiter auf der Seite der Produkte liegendes) Gleichgewicht ein.

Beispiele für derartige Gleichgewichtsreaktionen sind die Veresterung einer Carbonsäure mit einem Alkohol unter Bildung eines Alkylesters der Carbonsäure und Wasser gemäß der folgenden Reaktionsgleichung:

$$R^1CO\text{-}OH + R^2O\text{-}H \quad \rightleftarrows \quad R^1CO\text{-}OR^2 + H_2O \tag{1}$$

Durch kontinuierliches Abdestillieren des im Verlauf der Reaktion gebildeten Wassers oder durch Einsetzen eines der Edukte (Carbonsäure oder Alkohol) im Überschuß wird das Gleichgewicht auf die rechte (Produkt-) Seite der Reaktion verschoben. In vergleichbarer Weise lassen sich Carbonsäurealkylester in Gegenwart von Alkoholen unter Freisetzung eines Alkohols mit der primär im Ester gebundenen Alkoxygruppe umestern:

$$R^3CO\text{-}OR^4 + R^5O\text{-}H \quad \rightleftarrows \quad R^3CO\text{-}OR^5 + R^4O\text{-}H \tag{2}$$

Zur Beeinflussung der Lage des Gleichgewichts kann auch eine Änderung der Reaktionsbedingungen (Temperatur, Druck) beitragen. In der Praxis führen jedoch die genannten Maßnahmen mit dem Ziel einer Verschiebung des Gleichgewichts auf die Produktseite nicht immer zum gewünschten Erfolg. Dies ist regelmäßig darauf zurückzuführen, daß eine Änderung der Reaktionsbedingungen häufig auch die Bildung von Nebenprodukten mit sich bringt, was nicht nur im Hinblick auf die Produkt-Ausbeute, sondern auch auf die Reinheit der Produkte problematisch ist. Die Abtrennung der unerwünschten Nebenprodukte von den gewünschten Produkten kann - insbesondere in großtechnischen Verfahren - die Wirtschaftlichkeit erheblich beeinträchtigen. Die Präsenz großer Mengen an Edukt, das im Überschuß eingesetzt wurde, führt ebenfalls zu Problemen im Zusammenhang mit der Reinigung des gewünschten Produktes.

Physikalische Gleichgewichte können bei der Durchführung chemischer Reaktionen ebenfalls eine Rolle spielen.

So ist es häufig erforderlich, daß bestimmte Reaktionen in (z. B. flüssigen) Medien durchgeführt werden, die frei von Sauerstoff oder Wasser bzw. Feuchtigkeit sind, die in dem zur Reaktion vorgesehenen Medium physikalisch gelöst sind. Üblicherweise werden in solchen Fällen das das Medium enthaltende Gefäß evakuiert und anschließend mit einem Inertgas befüllt, oder das offene System wird über eine gewisse Zeit hinweg mit einem Inertgas gespült. Dadurch wird das unerwünschte Gas bzw. die Feuchtigkeit ausgetrieben; jedoch geht auch das Inert-Füllgas oder -Spülgas verloren. Zudem sind in Anlagen zur technischen Führung chemischer Reaktionen derartige Vorgänge kaum wirtschaftlich durchführbar.

Auch Lösungsgleichgewichte chemischer Stoffe in Lösungen spielen bei der technischen Führung chemischer Reaktionen eine Rolle. So können im Anschluß an eine chemische Reaktion unerwünschte Nebenprodukte oder Edukte in der Reaktionsmischung gelöst sein. Nebenprodukte wie beispielsweise Farb- oder Geruchsbildner oder niedermolekulare Nebenprodukte einer im statistischen Mittel zu höhermolekularen Produkten führenden Reaktion müssen genauso in aufwendigen und regelmäßig die Produktausbeute erheblich mindernden Reinigungsschritten entfernt werden wie in der Reaktionsmischung verbliebene, nicht umgesetzte Edukte.

Zu den Lösungsgleichgewichten - in diesem Fall diffusionsbestimmt - gehören auch die Gleichgewichte des Lösens gasförmiger Reaktionspartner in flüssigen Reaktionssystemen. Bei Gas-Flüssig-Reaktionen wie Hydrierungen, Oxidationsreaktionen, Nitrilierungen, Phosgenierungen oder Alkoxylierungen mit Alkylenoxiden bestimmt sich die Reaktionsgeschwindigkeit, d. h. die Geschwindigkeit, mit der sich ein möglichst weit auf der Produktseite liegendes Gleichgewicht einstellt, durch die Menge an im flüssigen Reaktionsmedium zum Kontakt mit dem gelösten Reaktionspartner verfügbarem Gas. Zur Erzielung praktikabler Reaktionsgeschwindigkeiten mußte der Partialdruck des Reaktionsgases in bisherigen Reaktionen relativ hoch sein, um eine ausreichende Gaskonzentration einstellen zu können. Dies machte nicht nur die Steuerung der Reaktion im Einzelfall schwierig, sondern erforderte regelmäßig den Einsatz größerer Gasmengen, als sie für die Reaktion tatsächlich erforderlich sind. Zum anderen konnte zum Schluß der Reaktion nicht verhindert werden, daß eine gewisse Gasmenge unumgesetzt wieder entfernt werden mußte, da deren Partialdruck im Gasraum nicht ausreichend für ein vollständiges Abreagieren des gasförmigen Reaktionspartners im flüssigen Reaktionsmedium in vertretbarer Reaktionszeit war. Letzteres ist insbesondere dann nicht vertretbar, wenn das Gas aus Gründen des Schutzes der Umwelt nicht abgelassen werden kann, sondern aufwendig entsorgt werden muß. Auch dies senkte die Wirtschaftlichkeit vieler Gas-Flüssig-Reaktionen.

Die Druckschrift EP 0 565 777 A1 beschreibt ein verbessertes Abstreifsystem, bei dem eine ringförmige Öffnung zwischen einer Venturi-Düse und einem konischen Mischer positioniert und automatisch anpaßbar ist, um Eigenschaftsänderungen in der Reaktionsmischung zu kompensieren. Dabei wird ein Gas nicht in einem Kreislauf geführt, sondern über eine Leitung angesaugt, durch die Reaktionsmischung geleitet und nach Hindurchtreten durch die Reaktionsmischung abgeblasen.

Der Erfindung lag die Aufgabe zugrunde, den oben beschriebenen Nachteilen des Standes der Technik abzuhelfen. Insbesondere sollte ein Verfahren zur Durchführung chemischer Reaktionen zur Verfügung gestellt werden, die durch Gleichgewichte gekennzeichnet sind, bei dem der Eingriff in das Gleichgewicht zugunsten des gewünschten Ablaufs der Reaktion in einfacher und effizienter Weise möglich ist und mit Mitteln erfolgen kann, die der gewünschten Verschiebung des Gleichgewichts keine physikalischen oder chemischen Kräfte entgegensetzen.

Eine weitere Aufgabe der Erfindung war, ein derartiges Verfahren zur Verfügung zu stellen, bei dem das Mittel zur Verschiebung des Gleichgewichts möglichst universell einsetzbar ist. Damit mußte das Mittel gegen chemische Reaktionen inert sein und im Hinblick auf eine wirtschaftliche Führung des Verfahrens auch preiswert und ubiquitär verfügbar sein.

Weitere Aufgaben, Vorteile und Merkmale des erfindungsgemäßen Verfahrens ergeben sich aus der nachfolgenden Beschreibung.

Überraschend wurde nämlich gefunden, daß es möglich ist, in einem Verfahren zur Durchführung von chemischen Reaktionen, die durch ein Gleichgewicht gekennzeichnet sind, ein Inertgas in einem separaten Gaskreislauf zu führen, wodurch es möglich ist, die gestellten Aufgaben zu lösen.

Die Erfindung betrifft ein Verfahren zur Durchführung einer durch ein Gleichgewicht gekennzeichneten physikalischen oder chemischen Reaktion, in einem als Schleifenreaktor ausgebildeten Reaktionssystem, umfassend einen Reaktorbehälter, mindestens eine mit dem Reaktorbehälter über je einen Auslaß und je einen Einlaß verbundene Schleife mit Einrichtungen zum Umpumpen des fluiden Reaktionsguts, mindestens einen Wärmetauscher, gegebenenfalls Einrichtungen zum Einspeisen des Reaktionsfluids in den Reaktorbehälter und einen getrennten Gaskreislauf, der mit dem über dem fluiden Reaktionsgemisch befindlichen Gasraum des Reaktorbehälters in Verbindung steht und getrennte Einrichtungen zum Einspeisen von Gas in den Gaskreislauf, zum Abziehen von Gas aus dem Gaskreislauf und zur Behandlung des im Gaskreislauf umlaufenden Gases aufweist, worin

ein Inertgas im Gaskreislauf geführt und behandelt wird, zum Eingreifen in die im Reaktorbehälter ablaufende, durch ein Gleichgewicht gekennzeichnete Reaktion in den Reaktorbehälter eingespeist wird und nach Eingriff in das Gleichgewicht der im Reaktorbehälter ablaufenden Gleichgewichtsreaktion aus dem Gasraum des Reaktorbehälters in den Gaskreislauf abgezogen wird, und worin

das Inertgas bei seinem Abziehen aus dem Reaktionsbehälter in den getrennten Gaskreislauf in der Weise in die durch ein Gleichgewicht gekennzeichnete Reaktion im Reaktorbehälter eingreift, daß es die Reaktionsmischung von darin gelöstem Gas und/oder von darin transportierten flüchtigen Stoffen befreit, indem es dieses/diese aus der Reaktionsmischung austrägt.

Im Rahmen der vorliegenden Erfindung wird unter dem Begriff "Gleichgewicht" sowohl ein physikalisches als auch ein chemisches Gleichgewicht verstanden. Es ergeben sich aus der nachfolgenden Beschreibung besondere Beispiele gemäß der Erfindung, in denen der Eingriff in das Gleichgewicht ein Eingriff in ein physikalisches Gleichgewicht (beispielsweise ein reines Lösungsgleichgewicht) ist oder in denen andererseits der Eingriff in das Gleichgewicht ein Eingriff in ein chemisches Gleichgewicht (beispielsweise einer Veresterung oder Umesterung) ist. Mitunter treten in einem Reaktionssystem auch Gleichgewichte beider Arten nebeneinander auf, in die gleichzeitig oder auch nacheinander eingegriffen werden kann.

Der für das erfindungsgemäße Verfahren verwendete Schleifenreaktor, der das Reaktionssystem umschließt, ist ein als solcher bekannter Schleifenreaktor. Solche Reaktoren werden üblicherweise als geschlossene Reaktionsbehälter zur Durchführung von Reaktionen von in flüssiger Phase gelösten Reaktionspartnern mit anderen in flüssiger Phase gelösten Reaktionspartnern einschließlich gasförmiger Reaktionspartner verwendet. Ein Beispiel einer derartigen, in einem Schleifenreaktor durchgeführten Reaktion ist in der Druckschrift EP-A 0 419 419 offenbart.

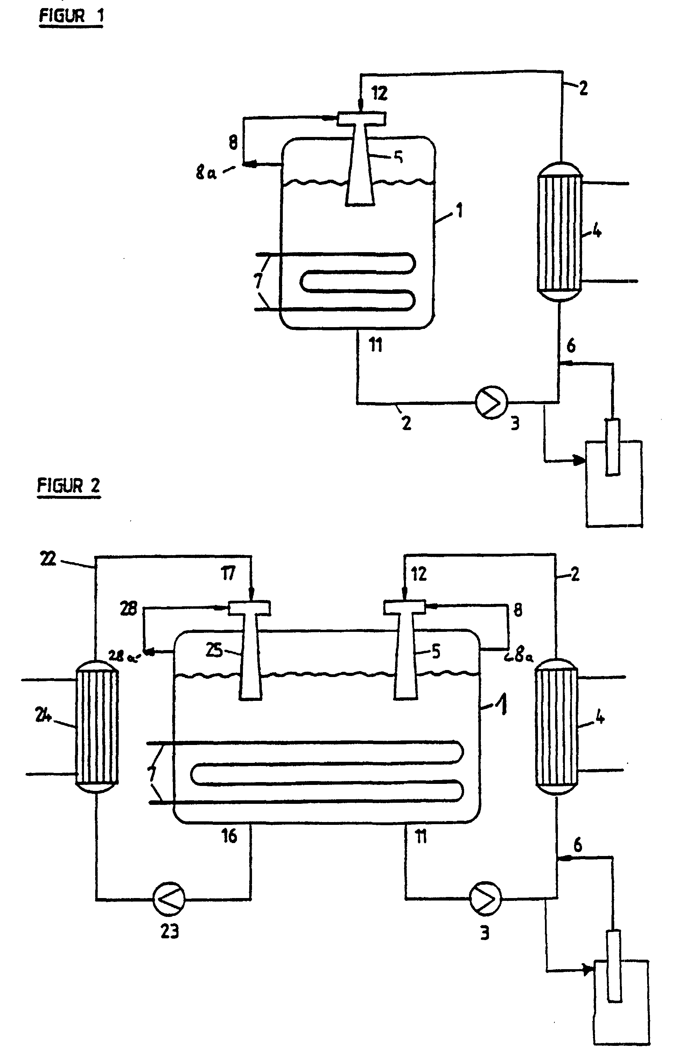

Derartige Schleifenreaktoren umfassen üblicherweise - wie aus Figur 1 ersichtlich - einen Reaktorbehälter (1), in dem sich ein erheblicher Teil, wenn nicht sogar die überwiegende Menge, der Reaktionslösung befindet. Dieser ist mit Vorrichtungen (7) zum Heizen und/oder Kühlen des Reaktionsguts ausgestattet, beispielsweise mit einem Mantel zur Führung von Gas oder Flüssigkeit, die einer Heizung oder Kühlung des Reaktionsguts dienen kann, sofern dies erforderlich ist. Mit dem Reaktorbehälter (1) verbunden über je einen Auslaß (11, 16) und je einen Einlaß (12, 17) ist mindestens eine Schleife (2, 22), durch die mittels Einrichtungen (3, 23) zum Umpumpen das fluiden Reaktionsgut zwangsweise geführt werden kann. Derartige Einrichtungen (3, 23) können beispielsweise Pumpen sein. In die Schleife (2, 22) eingebaut ist üblicherweise, im vorliegenden Fall bevorzugt, mindestens ein Wärmetauscher (4, 24), der im Rahmen der vorliegenden Erfindung je nach Reaktion, die in dem Schleifenreaktor durchgeführt wird, die Funktion eines Kühlers oder die Funktion einer Heizvorrichtung haben kann. Es ist auch möglich, daß bei zwei Schleifen (2, 22), wie sie beispielhaft in Figur 2 gezeigt sind, und - dementsprechend - zwei Wärmetauschern (4 und 24) zumindest zeitweise einer die Funktion eines Kühlers und einer die Funktion einer Heizvorrichtung übernimmt.

Am Einlaß der Schleife (2, 22) in den Reaktionsbehälter (1) kann eine Einrichtung zum Einspeisen des umlaufenden Reaktionsfluids in den Reaktorbehälter (1) angeordnet sein. Dies kann so geschehen, daß die Schleife (2, 22) am Einlaß in den Reaktorbehälter (1) das Reaktionsgut in eine solche Einrichtung einspeist oder der Einlaß selbst eine solche Einrichtung zum Einspeisen in den Reaktorbehälter darstellt. Beispiele hierfür sind Venturi-Rohre oder -Düsen, Strahldüsen oder Strahlsauger-Mischdüsen, die bei Einspeisen des Reaktionsgutes in den Reaktorbehälter (1) für eine gewisse Turbulenz sorgen und damit den Kontakt zwischen den Reaktionspartnern (insbesondere bei Gas-Flüssig-Reaktionen) erheblich verbessern.

Der Schleifenreaktor weist einen getrennten Gaskreislauf (8, 28) auf. Dieser steht mit dem über dem fluiden Reaktionsgut befindlichen Gasraum des Reaktorbehälters (1) in Verbindung und weist bevorzugterweise getrennte Einrichtungen zum Einspeisen von Gas in den Gaskreislauf (8, 28), getrennte Einrichtungen zum Abziehen von Gas aus dem Gaskreislauf (8, 28) und/oder getrennte und/oder getrennt abschaltbare oder zuschaltbare Einrichtungen zur Behandlung des im Gaskreislauf umlaufenden Gases auf. Diese Behandlung kann in bevorzugter Ausgestaltungen der Erfindung eine adsorptive Behandlung, eine kondensative Behandlung und eine selektive chemische (d. h. eine reaktive) Behandlung sein; Kombinationen daraus sind weiter bevorzugt und deswegen mit Vorteil einsetzbar, weil damit alle denkbaren Behandlungsmöglichkeiten nach Wahl zur Verfügung stehen.

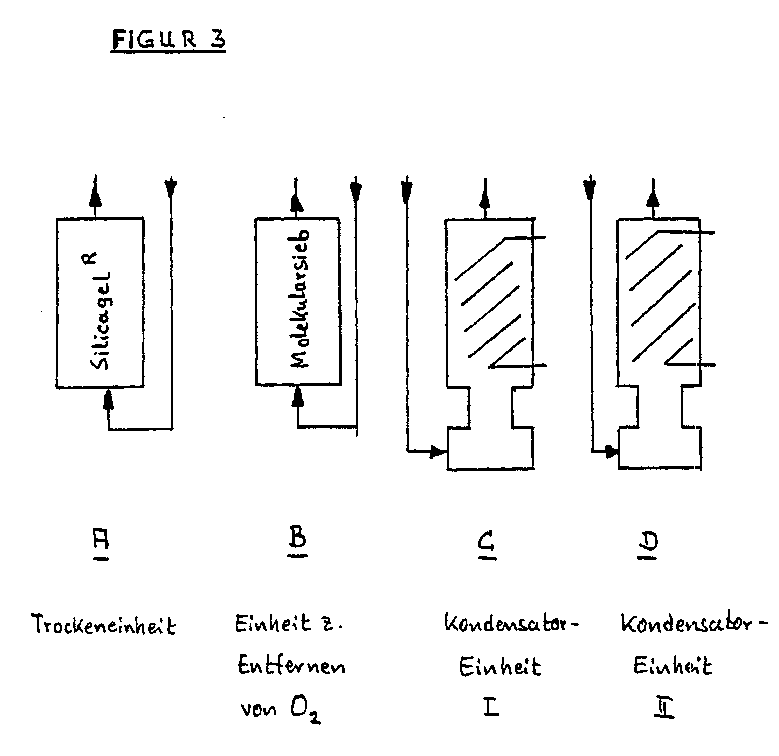

Die letztgenannten Einrichtungen sind in den Figuren 3A bis 3D gezeigt und können beispielsweise (bei 8a bzw. 28a in den Figuren 1 und 2) getrennt ab- und zuschaltbare Patronen oder Einheiten sein, die mit an sich bekannten Mitteln zum Entfernen von $O_2$ (z. B. mit einem Molekularsieb) oder zum Entfernen von Feuchtigkeit (z. B. mit einem Trocknungsmittel wie beispielsweise Silicagel[R]), mit einem Adsorptionsmittel wie z. B. Aktivkohle z. B. zum Zweck der Entfernung von niedermolekularen flüchtigen Komponenten oder auch mit einem Träger, der durch aktive chemische Reaktion Reaktivkomponenten selektiv bindet und damit entfernt bzw. in ungefährliche, nichttoxische und/oder nicht umweltrelevante Stoffe überführt, gefüllt sind. Alternativ können derartige Einrichtungen auch beispielsweise getrennt ab- und zuschaltbare Patronen bzw. Aggregate oder Einheiten in Form eines oder mehrerer, gegebenenfalls hintereinanderschaltbarer, Kondensatoren sein, mit denen Komponenten, die mit dem Inertgas aus dem Reaktionssystem in den Gaskreislauf ausgeschleppt werden, von dem Inertgas (gegebenenfalls einzeln bzw. in Stufen) abgetrennt werden können. Auch Kombinationen derartiger Einrichtungen (also z. B. eine Trockenpatrone und ein oder mehrere Kondensator(en) sind erfindungsgemäß möglich.

In einem solchen Schleifenreaktor wird erfindungsgemäß ein Inertgas im Gaskreislauf (8, 28) geführt und/oder behandelt, zum Eingreifen in die im Reaktorbehälter (1) ablaufende, durch ein Gleichgewicht gekennzeichnete Reaktion in den Reaktorbehälter (1) eingespeist und nach Eingriff in das Gleichgewicht der im Reaktorbehälter (1) ablaufenden Gleichgewichtsreaktion aus dem Reaktorbehälter (1) in den Gaskreislauf (8, 28) abgezogen.

Als Inertgase können eine Vielzahl von Gasen infrage kommen. Grundsätzlich sind alle Gase geeignet, die in der speziellen Reaktion inert sind, d. h. nicht mit einem der Reaktionspartner in unerwünschter Weise reagieren. Im Sinne dieser allgemeinen Definition von Inertgas kann für eine spezielle Gleichgewichtsreaktion ein Gas verwendet werden, das unter den gegebenen Reaktionsbedingungen nicht mit einem der Reaktionspartner in unerwünschter Weise reagiert, jedoch in anderen Reaktionen oder unter anderen Reaktionsbedingungen nicht inert ist. Im Sinne einer möglichst universellen Verwendbarkeit werden jedoch erfindungsgemäß bevorzugt Stickstoff ($N_2$), Argon (Ar), Kohlendioxid ($CO_2$), Wasser ($H_2O$) oder sonstige inerte, bei den Reaktionsbedingungen gasförmige Stoffe als Inergase verwendet. Auch Mischungen eines der genannten Gase mit einem oder mehreren anderen Inertgasen sind erfindungsgemäß verwendbar. Es ist einer der Vorteile der Erfindung, daß in dem geschlossenen System des Schleifenreaktors auch solche Inertgase im industriellen Maßstab eingesetzt werden können, die sonst aus Kostengründen nicht infrage kommen, da das Reaktionssystem lediglich ein einziges Mal mit dem gewünschten Inertgas befüllt zu werden braucht und dieses im Gaskreislauf (8, 28) und im Gasraum über dem fluiden Reaktionsmedium im Kreislauf geführt und gegebenenfalls auch gereinigt und danach wieder in den Kreislauf zurückgeführt wird. Ein Verlust auch teurer Inertgase kann dadurch vermieden werden. Besonders bevorzugt wird jedoch als Inertgas Stickstoff verwendet, da er ubiquitär verfügbar ist, preiswert gewonnen werden kann und hinsichtlich seiner Eigenschaften, Einfluß auf Gleichgewichte zu nehmen, wirkungsvoll ist.

Das Inertgas wird im Gaskreislauf (8, 28) geführt und/oder behandelt. Die Führung im Gaskreislauf (8, 28) kann also dazu dienen, das Inertgas für den späteren Eingriff in das Gleichgewicht der im Reaktionssystem ablaufenden Reaktion zu konditionieren. Neben der Befreiung des Inertgases von unerwünschten Komponenten (z. B. Feuchtigkeit = Trocknung; Sauerstoff bzw. Luft = Entfernung von Sauerstoff; andere gasförmige Begleitstoffe = Reinigung) kann eine derartige Konditionierung in bevorzugten Ausführungsformen des erfindungsgemäßen Verfahrens in einer Erhitzung, Abkühlung, oder Beaufschlagung des Inertgases mit erwünschten gasförmigen Stoffen liegen. Letzteres kann beispielsweise von Vorteil sein, wenn der erwünschte gasförmige Stoff zur besseren Verteilung trotz Senkung seines Partialdrucks im System in das fluide Reaktionssystem eingetragen werden soll, um das Gleichgewicht im Sinne einer Verbesserung des Kontakts des gasförmigen Stoffes mit dem/den anderen Reaktionspartner(n) zu beeinflussen. Eine weitere vorteilhafte Ausgestaltung der Beaufschlagung des Inertgases mit einem erwünschten gasförmigen Stoff liegt in der gezielten Schaffung eines reduktiven oder oxidativen Milieus während einer ansonsten nicht redox-beeinflußten Reaktion. Dies kann beispielsweise erwünscht sein, um Nebenreaktionen zu vermeiden.

Erfindungsgemäß wird das Inertgas aus dem separaten Gaskreislauf (8, 28), in dem es geführt und/oder behandelt wird, in den Reaktorbehälter (1) eingespeist, um in das im Reaktionsmedium herrschende Gleichgewicht einzugreifen bzw. um das Gleichgewicht im gewünschten Sinn zu beeinflussen. Dabei sind verschiedene Formen des Eingriffs bzw. der Einflußnahme möglich, die nachfolgend im einzelnen erläutert werden. Die Einspeisung des Inertgases kann dabei sowohl in den Gasraum über dem fluiden Reaktionsmedium oder direkt in das fluide Reaktionsmedium erfolgen. Im letztgenannten Fall kann das Inertgas beispielsweise über die Einrichtung (5, 25) zum Einspeisen des fluiden Reaktionsmediums in den Reaktorbehälter (1) eingespeist werden. Es sind jedoch auch andere Wege des Einspeisens möglich, beispielsweise direkt in die Schleife (2, 22) oder an anderer Stelle in das umlaufende Reaktionsgut. Dies ist im Einzelfall der Wahl des Fachmanns auf diesem Gebiet überlassen.

Das Inertgas greift in der Weise in die durch ein Gleichgewicht gekennzeichnete Reaktion im Reaktorbehälter (1) ein, daß das Inertgas die Reaktionsmischung von darin gelöstem Gas (oder auch unerwünschten niedermolekularen Bestandteilen, die sonst nur durch eine separate Trägerdampf-Destillation entfernt werden können) reinigt und dieses aus der Reaktionsmischung austrägt. Der Eingriff in das Gleichgewicht

$$\text{Reaktionsmischung + darin gelöstes Gas} \rightleftarrows \text{Reaktionsmischung + Gas } (\uparrow) \qquad (3)$$

führt dazu, daß eine von unerwünschten gasförmigen Komponenten befreite Reaktionsmischung entsteht. Dies kann beispielsweise dann erforderlich sein, wenn eine Realctionsmischung keinen Sauerstoff enthalten darf, weil dieser die Reaktion stört, in eine unerwünschte Richtung lenkt oder eine der an der Reaktion beteiligten Komponenten hinsichtlich ihrer Wirkungsweise in der Reaktion beeinträchtigt. Eine derartige Befreiung des Reaktionsmediums von Sauerstoff wird beispielsweise vor der Reaktion durchgeführt zum Schutz von Reaktionsprodukten vor oxidativem Abbau (z. B. bei der Kondensation von hoch-ungesättigten Fettsäuren oder bei der Veresterung hoch-ungesättigter Verbindungen), zur Vermeidung der Bildung von niedermolekularen Nebenprodukten (z. B. bei Polymerisationsreaktionen) oder zum Schutz von Katalysatoren gegen Wirkungsverlust (z. B. zum Schutz von phosphoriger Säure gegen Oxidation zu Phosphorsäure bei der Veresterung von Sorbit zu Sorbitanestern). Dazu trägt man das Inertgas wie beispielsweise Stickstoff, das im Gaskreislauf (8, 28) geführt wird, bevorzugt direkt in das fluide Reaktionsgut ein. Das Inertgas durchläuft beispielsweise (aber nicht notwendigerweise) das Reaktionsgut unter hoher Turbulenz, beispielsweise in Form einer Vielzahl möglichst kleiner Blasen, und überlagert das Gleichgewicht (3) mit dem folgenden Gleichgewicht (4):

$$\text{gelöstes Gas + N}_2 \rightleftarrows \text{gelöster N}_2 + \text{Gas } (\uparrow) \qquad (4)$$

Das aus der Lösung durch eingetragenen Stickstoff "verdrängte" Gas wird mit überschüssigem Stickstoff aus der Reaktionsmischung und anschließend aus dem Reaktionsbehälter (1) ausgetragen, gelangt in den Gaskreislauf (8, 28) und wird dort im Zuge der Behandlung des Inertgases wieder vom Inertgas getrennt. Dabei kann die Behandlung der Inertgas-Sauerstoff-Mischung im Gaskreislauf nach dem Fachmann für eine solche Behandlung bekannten Verfahrensweisen durchgeführt werden. Eine solche Verfahrensweise kann - um ein Beispiel zu nennen - die Behandlung der Inertgas-Sauerstoff-Mischung mittels eines Molekularsiebes sein, das den Sauerstoff selektiv entfernt. Dieses ist vorteilhafterweise (aber nicht zwingend) in eine Patrone gefüllt, durch die das Gasgemisch zur Behandlung strömt. Andere Verfahrensweisen sind in gleicher Weise denkbar, um den Sauerstoff vom Inertgas zu trennen. Das Inertgas steht dann wieder für einen erneuten Zyklus der Entfernung von Sauerstoff zur Verfügung. Verluste an Inertgas treten nicht auf. Selbstverständlich kann auch in einem offenen System mit Stickstoff der Sauerstoff ausgespült werden, ohne den Stickstoff wieder in den Kreislauf zurückzuführen. Diese Ausführungsform ist jedoch gegenüber der vorher beschriebenen weniger bevorzugt.

Es ist eine weitere Ausprägung desselben Prinzips, in einem der eigentlichen Reaktion vorgelagerten Schritt durch gezielten Zusatz geringer Mengen eines reaktiven Gases die Reaktionsmischung so zu konditionieren, daß in der anschließenden Reaktion die Bildung unerwünschter Nebenprodukte verhindert wird. So können z. B. vor Reaktionen ungesättigter Roh-Fettsäuren oxidativ gebildete Zentren (Hydroperoxide, Peroxide und andere Zwischenstufen) durch Vorbehandlung mit $H_2$ reduziert werden.

In gleicher Weise kann das Inertgas vor Beginn der Reaktion im Reaktionsbehälter dazu verwendet werden, Feuchtigkeit aus der für die Reaktion vorgesehenen Reaktionsmischung auszutreiben. Im Zuge dieses Eingriffs des Inertgases in das Lösungsgleichgewicht wird das Inertgas, beispielsweise Stickstoff, wie im vorstehend beschriebenen Fall in die Reaktionsmischung bevorzugt turbulent eingetragen, verdrängt das gelöste Wasser aus der Reaktionsmischung und schleppt dieses in den Gasraum über der Reaktionsmischung bzw. in den Gaskreislauf (8, 28) aus. Im Gaskreislauf wird das Inertgas dann in an sich bekannter Weise "getrocknet", d. h. das Wasser wird abgetrennt. Dies geschieht im Gaskreislauf in an sich bekannter Weise. Ein Beispiel für den Trocknungsvorgang, der dann besonders bevorzugt ist, wenn geringe Mengen Wasser (Feuchtigkeit) durch das Inertgas ausgeschleppt werden, besteht darin, das feuchte Inertgas eine Patrone mit einem üblichen Trocknungemittel durchströmen zu lassen, beispielsweise eine Patrone mit Silicagel$^R$. Bei größeren Wassermengen sind andere, dem Fachmann bekannte Verfahren wie das Ausfrieren von Wasser in einem in den Gaskreislauf eingeschalteten Kühler bevorzugt. Nach dem "Trocknen" steht das Inertgas für einen weiteren Zyklus zur Verfügung. Verluste an Inertgas treten nicht auf. Selbstverständlich kann auch in einem offenen System mit Stickstoff die Feuchtigkeit (das Wasser) ausgespült werden, ohne den Stickstoff wieder in den Kreislauf zurückzuführen. Diese Ausführungsform ist jedoch gegenüber der vorher beschriebenen weniger bevorzugt.

Selbstverständlich ist es in dem erfindungsgemäßen Verfahren bei der Behandlung des Gases im Gaskreislauf möglich, Einheiten zur Kondensation, Einheiten zur Adsorption, Einheiten zur Trocknung, Einheiten zum reaktiven Abreagieren von Reaktionskomponenten usw. in beliebiger (jedoch für die Verfahrensführung sinnvollen) Reihenfolge und Kombination hintereinanderzuschalten. Damit kann in vorteilhafter Weise der Abtrenngrad der bei der Behandlung im Gaskreislauf abzutrennenden Komponenten erhöht werden, was die Gesamteffizienz des Verfahrens verbessert. Noch weiter bevorzugt ist es, große Mengen an einem Gleichgewicht entzogenen und in den Gaskreislauf ausgeschleppten flüchtigen Komponenten kondensativ abzutrennen und danach kleinere (Rest-) Mengen adsorptiv oder durch chemische Reaktion abzutrennen.

Die gleiche Verfahrensweise, wie sie vorstehend beschrieben wurde, findet Anwendung, wenn aus dem Reaktionssystem bereits im Roh-Edukt vorliegende niedermolekulare Verunreinigungen entfernt werden sollen, die sich durch Alterung und/oder Zersetzung gebildet hatten. Diese müssen bei herkömmlicher Vorgehensweise mühsam durch eine Trägerdampfdestillation entfernt werden. Das der vorliegenden Erfindung zugrundeliegende Verfahren ermöglicht eine einfachere und schonendere Entfernung derartiger Verunreinigungen bereits im System, in dem die spätere Reaktion stattfindet.

Eingriffe des Inertgases in das Lösungsgleichgewicht finden in einer weiteren bevorzugten Ausführungsform des endungsgemäßen Verfahrens auch dann statt, wenn eine mit einem Gas als Reaktionspartner stattfindende Gleichgewichtsreaktion durch Zufuhr eines Gas-Überschusses auf die Seite der Produkte verschoben wird. Nach der obigen Reaktion (4) kann der nicht umgesetzte Gas-Überschuß aus der Reaktionsmischung ausgetragen und damit bereits im Reaktionsgefäß eine schnelle, effiziente und vollständige Reinigung von überschüssigem gasförmigem Reaktionspartner erreicht werden. Dies spielt im industriellen Bereich beispielsweise bei batchartig geführten Verfahren eine Rolle, insbesondere bei Reaktionen mit umweltrelevanten, toxischen oder hochreaktiven Gasen. Beispiele hierfür sind Phosgenierungen, Carbonylierungsreaktionen oder Alkylierungen. Die durch das Inertgas ausgetragenen überschüssigen Reaktionspartner (z. B. $COCl_2$, CO oder RCl) werden nach an sich bekannten Verfahren von dem Inertgas abgetrennt, z. B. reaktiv oder durch Adsorption oder Kondensation.

In einer weiteren bevorzugten Ausführungsform des erfindungsgemäßen Verfahrens greift das Inertgas in der Weise in die durch ein Gleichgewicht gekennzeichnete Reaktion im Reaktorbehälter (1) ein, daß die Behandlung des Inertgases im Gaskreislauf neben der Befreiung des Inertgases von darin enthaltenen Komponenten aus dem Reaktionsgemisch zusätzlich in einer nachfolgenden Beaufschlagung des Inertgases mit flüchtigen Stoffen für das Reaktionsgemisch besteht. Dies kann in allen Fällen zu positiven Ergebnissen führen, in denen einer Reaktionsmischung gasförmige Reaktionspartner zugeführt werden müssen und

- entweder eine beschleunigende oder bremsende Steuerung der Reaktion erwünscht ist;
- oder (gegebenenfalls auch im Hinblick auf eine Steuerung der Reaktion) ein bestimmter, beispielsweise niedriger Partialdruck des gasförmigen Reaktionspartners erwünscht ist;
- oder ein Gleichgewicht "sanft" und unter Vermeidung hoher Überschüsse eines gasförmigen Reaktionspartners zu den Produkten verschoben werden soll.

Der letztgenannte, relativ häufige Eingriff in ein Gleichgewicht wird beispielsweise bei chemischen Reaktionen notwendig, die - natürlich unerwünscht - erst nach einer gewissen Inkubationszeit anlaufen ("anspringen"). Die Inkubationszeit wird dann, wenn das erfindungsgemäße Verfahren unter Eingriff in das Gleichgewicht zur Anwendung kommt, überraschenderweise verkürzt oder entschärft, wenn nicht sogar vermieden.

Beispiele der oben genannten Reaktionen können alle unter Gasbeteiligung auf der Eduktseite ablaufenden Gleichgewichtsreaktionen sein, beispielsweise Hydrierungen, Oxidationsreaktionen, Alkoxylierungsreaktionen, Nitrilierungen, Phosgenierungen, Halogenierungen Alkylierungen, Sulfierungen, Sulfatierungen etc.. Bei solchen Reaktionen kann es erwünscht sein, durch gemeinsamen Eintrag des Reaktionsgases mit dem Inertgas einen besseren Kontakt zwischen dem Reaktionsgas und dem bzw. den in der Reaktionsmischung bereits gelösten Reaktionspartner(n) herzustellen, eine zu schnelle Reaktion bei hochreaktiven gasförmigen Reaktionspartnern unter Vermeidung von Nebenreaktionen bei zu hoher Temperatur oder zu hoher Konzentration des Reaktionsgases durch "Verdünnung" des Reaktionsgases zu vermeiden, einen zu starken Überschuß eines Reaktionsgases aus wirtschaftlichen oder reaktionstechnischen Gründen zu vermeiden oder gesundheitsgefährdende oder gefährliche oder teure Reaktionsgase in die Reaktion nur bei niedrigen Partialdrücken, doch nicht minder effizient einzusetzen. Diesen Zielen trägt der Eintrag des Reaktionsgases mit dem Inertgas gemäß der Erfindung Rechnung.

Wesentliche Vorteile lassen sich insoweit erzielen, als die thermischen wie kinetischen und thermodynamischen Parameter der Reaktionsführung verbessert werden. Außerdem werden durch die "Verdünnung" des Reaktionsgases bei Erhaltung der Reaktivität in Bezug auf die flüssige Phase Nebenreaktionen unter Beteiligung des Reaktionsgases vermieden oder weitgehend unterdrückt. So sind u. a. die Bildung von Zersetzungsprodukten oder auch Kondensations- oder Umlagerungsreaktionen (Inaktivierung) vermeidbar.

Es entspricht einer besonders bevorzugten Ausführungsform der Erfindung, daß der durch das Inertgas zugeführte gasförmige Reaktionspartner in durch das Auslaufen der Reaktion bedingtem niedrigem Partialdruck vorliegt. Dies spielt beispielsweise eine Rolle, wenn eine unter Beteiligung eines gasförmigen Reaktionspartners ablaufende Gleichgewichtsreaktion nicht abrupt abgebrochen wird, sondern nach Beendigung der Zufuhr des gasförmigen Reaktionspartners unter mehr oder weniger vollständiger Abreaktion des Gases zum Abschluß kommen soll, wie z. B. bei Alkoxylierungsreaktionen. Im Stand der Technik blieb regelmäßig eine bestimmte Gasmenge (Alkylenoxid) unumgesetzt in der Reaktionsmischung bzw. im Gasraum des Schleifenreaktors, wenn man die Reaktion nicht unwirtschaftlich lange weiterlaufen lassen wollte. Erfindungsgemäß kann unter Verwendung eines Inertgases wie $N_2$ selbst bei am Ende der Reaktion zurückgehendem Partialdruck des gasförmigen Reaktionspartners ein vollständiges Abreagieren erreicht werden, und es muß kein Reaktionsgas abgelassen oder abgepumpt werden. Dadurch werden nicht nur separate Maßnahmen zur Vermeidung einer Gefährdung (Sicherheit, Gesundheit) der Umwelt vermieden (z. B. aufwendige Gaswäscher), sondern auch eine wirtschaftliche Verfahrensführung unter Ausnutzung der Ressourcen ermöglicht.

Eine große Anwendungsbreite im Sinne einer Beeinflussung von Gleichgewichtsreaktionen ergibt sich in der erfindungsgemäß bevorzugten Ausführungsform, in der das Inertgas in der Weise in die durch ein Gleichgewicht gekennzeichnete Reaktion im Reaktorbehälter (1) eingreift, daß das Inertgas der Reaktionsmischung einen flüchtigen Reaktionspartner entzieht. Dabei ist besonders bevorzugt, daß der Entzug des flüchtigen Reaktionspartners durch das Inertgas während des Verlaufs der Reaktion erfolgt und der durch das Inertgas der Reaktionsmischung entzogene Reaktionspartner ein im Verlauf der Reaktion entstehendes Produkt der durch ein Gleichgewicht gekennzeichneten Reaktion ist. So kann beispielsweise bei der oben durch Gleichung (1) wiedergegebenen Veresterungsreaktion das flüchtige Reaktionsprodukt Wasser durch ein Inertgas aus dem Gleichgewicht ausgetragen werden. Damit wird das Gleichgewicht der Veresterungsreaktion ohne Temperaturbelastung und ohne Einsatz von Edukt-Überschuß wirksam auf die Produktseite verschoben. Das Wasser wird in einfacher Weise aus der Reaktionsmischung ausgetragen und in den Gaskreislauf ausgeschleppt. Dort läßt es sich problemlos auf an sich bekannte Weise von dem Inertgas abtrennen, das danach für einen erneuten Entwässerungszyklus zur Verfügung steht. Es treten keine Verluste an Inertgas auf.

In analoger Weise kann im Verlauf der durch die obige Reaktionsgleichung (2) wiedergegebenen Umesterungsreaktion der als Produkt entstehende Alkohol $R^4$O-H als flüchtiges Produkt aus der Reaktionsmischung ausgetragen und damit das Gleichgewicht der Umesterungsreaktion gewünschtenfalls auf die Produktseite verschoben werden. Die Abtrennung des Alkohols von dem Inertgas im Zuge der Behandlung des Inertgases im Gaskreislauf (8, 28) ist genauso unproblematisch (und an sich bekannt) wie die Abtrennung von Wasser nach der Veresterungsreaktion. Letzten Endes wird wieder reines Inertgas für einen erneuten Zyklus erhalten.

Auch das Gleichgewicht einer gerichteten Umesterung nach der Reaktionsgleichung (5)

$$R^6\text{CO-O}R^7 + R^8\text{CO-O}R^9 \rightleftarrows R^6\text{CO-O}R^9 \ (\uparrow) + R^8\text{CO-O}R^7 \qquad (5)$$

kann während des Verlaufs der Reaktion durch Zugabe eines Inertgases in Richtung auf die Produkte verschoben werden. Dies ist insbesondere dann möglich, wenn das Reaktionsprodukt $R^6$CO-O$R^9$ aufgrund der Länge der Alkylketten der Reste $R^6$ und $R^9$ flüchtig ist und damit durch das Inertgas ausgetragen werden kann. Technisch spielt dies beispielsweise eine Rolle bei der Umesterung von Kokosfett zur Erhöhung von dessen Schmelzpunkt: Dabei werden Ester relativ langkettiger Fettsäuren mit Kokosfett umgesetzt und langkettige Acylreste in das Fett eingeestert. Hierbei werden naturgemäß kürzerkettige Fettsäuren in Form ihrer Ester frei. Das Gleichgewicht der Reaktion läßt sich durch Abtreiben der relativ kurzkettigen Ester auf die Produktseite verschieben. Das Inertgas trägt die Ester der relativ kürzerkettigen Fettsäuren in den Gaskreislauf aus, wo sie bei der Behandlung des Inertgases abgetrennt werden. Das Inertgas steht anschließend für einen weiteren Zyklus zur Verfügung.

Die prinzipiell gleiche Reaktion findet statt bei der Umesterung von Triglyceriden mit nichtflüchtigen Fettsäuren oder der Umesterung von Polyalkoholen mit Methylestern: Auch bei diesen Reaktionen läßt sich das Gleichgewicht bei Ausschleusen der gebildeten flüchtigen Fettsäuren bzw. des Methanols auf die Seite der Produkte verschieben.

In weiteren bevorzugten Ausführungsformen des erfindungsgemäßen Verfahrens greift das Inertgas in der Weise unter Entzug eines flüchtigen Reaktionspartners in ein Reaktionsgleichgewicht ein, daß der Eingriff nach vollständigem Ablauf der Reaktion erfolgt und entweder der durch das Inertgas der Reaktionsmischung entzogene Reaktionspartner ein im Überschuß eingesetztes Edukt der durch ein Gleichgewicht gekennzeichneten Reaktion ist oder der durch das Inertgas der Reaktionsmischung entzogene Reaktionspartner ein im Verlauf der Reaktion entstandenes Produkt der durch ein Gleichgewicht gekennzeichneten Reaktion ist oder alternativ dazu ein Reaktionspartner ein im Überschuß eingesetztes Edukt der durch ein Gleichgewicht gekennzeichneten Reaktion ist und ein anderer ein im Verlauf der Reaktion entstandenes Produkt der durch ein Gleichgewicht gekennzeichneten Reaktion ist.

Ein Beispiel für den ersten Fall (der durch das Inertgas der Reaktionsmischung entzogene Reaktionspartner ist ein im Überschuß eingesetztes Edukt der Reaktion) ist die Desodorierung der Reaktionsmischung nach der Umsetzung von Glycerin und unter Verfahrensbedingungen entfernbarer, gegebenenfalls kurzkettiger Fettsäure zu Triglyceriden nach der Gleichung (6)

$$\text{Glycerin} + 3\, R^{10}\text{CO-OH} \rightleftarrows \text{Triglycerid} + 3\, H_2O \qquad\qquad (6)$$

Da schon geringe Mengen nicht umgesetzter, stark riechender Fettsäure zu einer erheblichen Geruchs- und Geschmacksbeeinträchtigung des Produktes führen, ist eine Desodorierung der Produktmischung nach Abschluß der Reaktion essentiell. Aus der Produktmischung kann die relativ flüchtige Fettsäure durch Einsatz eines Inertgases problemlos ausgetrieben werden, wodurch eine Desodorierung des Produktes im industriellen Maßstab ohne aufwendige Zusatzschritte schnell und effizient durchgeführt werden kann. Ebenso kann bei Einsatz eines Überschusses Glycerin dieser Überschuß nach Abreaktion der Fettsäure entfernt werden. Gegebenenfalls werden überschüssige Edukte wieder in den Kreislauf zurückgeführt.

Beispiele für den zweiten Fall (der durch das Inertgas der Reaktionsmischung entzogene Reaktionspartner ist ein im Verlauf der Reaktion entstandenes Produkt der durch ein Gleichgewicht gekennzeichneten Reaktion) sind die nach der obigen Gleichung (1) ablaufende Veresterung oder die nach der obigen Gleichung (2) ablaufende Umesterung: Mit Hilfe eines Trägergases wie Stickstoff wird der gewünschte Ester ($R^1$CO-OR$^2$ im Fall der Reaktion (1); $R^3$CO-OR$^5$ im Fall der Reaktion (2)), soweit dieser flüchtig ist, aus der Produktmischung abgetrennt und damit ein reines, von Nebenprodukten und/oder Edukten freies Produkt in einem Schritt erhalten. Das Produkt wird bei der Behandlung des Inertgases auf an sich bekannte Weise restlos von dem Inertgas abgetrennt und letzten Endes wieder ein reines, für einen neuen Trennzyklus geeignetes Inertgas erhalten.

Beispiele für den dritten Fall (ein Reaktionspartner ist ein im Überschuß eingesetztes Edukt der durch ein Gleichgewicht gekennzeichneten Reaktion und ein anderer ist ein im Verlauf der Reaktion entstandenes Produkt der durch ein Gleichgewicht gekennzeichneten Reaktion) sind die Anreicherung von Produkten eines bestimmten Alkoxylierungsgrades bei der Alkoxylierung von beispielsweise Fettalkoholen und die Reinigung der durch Umsetzung von Glycerin und beispielsweise Fettsäuremethylestern entstehenden Triglyceride: Bei der Alkoxylierung können nach Abschluß der Reaktion die relativ flüchtigen Reaktionspartner Fettalkohol (Edukt) und niedrig alkoxylierte Fettalkohole (Produkte) durch Eingriff eines Inertgases in das Lösungsgleichgewicht von den höher alkoxylierten Fettalkoholen (Produkte) abgetrennt und damit die gewünschten Produkte in relativ hoher Ausbeute und Reinheit erhalten werden. Die mit dem Inertgas abgezogenen Substanzen werden im Inertgas-Behandlungsschritt leicht wieder abgetrennt. In vergleichbarer Weise lassen sich bei der Triglycerid-Herstellung eingesetzter Fettsäuremethylester (Edukt) wie auch entstandenes Methanol (Produkt), gegebenenfalls entstandene Partialester des Glycerins (Monoester oder Diester) bzw. entstandener Methylether (Nebenprodukt) als leichter flüchtige Bestandteile der Reaktionsmischung nach Abschluß der Reaktion abtrennen.

In einer besonders bevorzugten Ausführungsform der Erfindung findet das vorstehend beschriebene Prinzip Anwendung bei der Desodorierung von Nahrungsmittelfetten bzw. -ölen. Diese waren im Stand der Technik in Kaskaden unter Vakuum (ca. 1,33 mbar) bei Temperaturen bis zu 270 °C im Gegenstrom gegen Wasserdampf behandelt worden, um unerwünschte Nebenprodukte mit einem Molekulargewicht unter dem der Fette bzw. Öle auszutreiben. Gleichzeitig erfolgte auch zum Teil eine Zersetzung von Farbkörpern und Epoxiden bzw. Hydroperoxiden aus der chemischen Bleichung bzw. adsorptiven Bleichung mittels Bleicherden und deren anschließende Entfernung. Im erfindungsgemäßen Verfahren erfolgt die Beseitigung der Nebenprodukte, d. h. eine Bleichung und anschließende Desodorierung, im Schleifenreaktor mittels Eintrag von $H_2$ mit $N_2$ und gegebenenfalls in Gegenwart eines Katalysators und anschließend unter Eintrag von $N_2$ zur Ausschleusung der Nebenprodukte in den Gaskreislauf (gegebenenfalls unter Zusatz von Wasserdampf zur Verbesserung der Abtrennwirkung). Der Stickstoff trägt die leicht flüchtigen Geruchsbildner in den Gaskreislauf aus, wo sie auf dem Fachmann bekannte Weise, beispielsweise adsorptiv unter Einschaltung einer ein Adsorptionsmittel enthaltenden Patrone, vom "Schleppgas" abgetrennt werden. Eine derartige Desodorierung bietet sich insbesondere nach der industrieüblichen Hydrierung von Speisefetten bzw. -ölen an, die ebenfalls im Schleifenreaktor mit Vorteil durchgeführt werden kann, wobei das erfindungsgemäße Verfahren die im Rahmen der Beschreibung aufgezeigten Vorteile mit sich bringt. Besonders vorteilhaft ist, daß bisher verwendete Anlagen dazu nur mit dem außerhalb des Reaktorsystems liegenden Gaskreislauf nachgerüstet zu werden brauchen.

Die letztgenannten Reaktionen werden - wie beschrieben - regelmäßig nach Abschluß der Reaktion durchgeführt, und zwar meist schon deswegen, weil der Gehalt an Edukt während der Reaktion benötigt wird, um das Gleichgewicht nicht unerwünschtermaßen wieder in Richtung auf die Edukte zu verschieben. Es ist jedoch auch im Einzelfall möglich, die Abtrennung der Edukte und/oder Abtrennung der entstandenen Reaktionsprodukte bereits im Verlauf des Verfahrens durchzuführen, wenn sich damit für die Lage des Gleichgewichts oder für andere Merkmale der Reaktion Vorteile ergeben.

Es entspricht einer weiteren, im Rahmen des erfindungsgemäßen Verfahrens bevorzugten Ausführungsform, mit Hilfe des Inertgases eine bestimmte Gasatmosphäre über der Reaktionsmischung und damit auch in der Reaktionsmischung einzustellen und damit den Ablauf von Reaktionen zu begünstigen. Dies kann beispielsweise von Bedeutung sein bei der Behandlung von natürlichen Fetten vor chemischen Reaktionen: Im Fett durch die Lageroder Transportbedingungen vorhandene reduzierbare Zentren (Epoxid-Gruppen; Hydroperoxid-Gruppen) können zur Vorbehandlung durch Eintrag von $H_2$ reduziert werden. Dazu wird ein Inertgas als Träger bzw. Medium zum Eingriff in das Lösungs- und Reaktionsgleichgewicht verwendet. Die Effizienz der Reaktion wird deutlich verbessert, und die Folgereaktionen laufen unter vollständigem Erhalt des gewünschten Fettproduktes ab.

Die Einstellung reduzierender Bedingungen ist auch bei der Veresterung von Sorbit zu Sorbitanestern erwünscht: Für diese (im wesentlichen nach Gleichung (1) ablaufende) Reaktion werden Katalysatoren (phosphorige Säure und deren Salze) gewählt, die reduzierende Eigenschaften haben, um die Bildung von die Qualität der Produkte beeinträchtigenden farbgebenden Nebenprodukten zu verhindern. Setzt man übliche Veresterungskatalysatoren unter reduzierenden Bedingungen ein ($H_2$-Eintrag in die Reaktionsmischung mittels des inerten Trägergases $N_2$), wird die Bildung von Nebenprodukten, insbesondere von farbgebenden und geruchsbildenden Substanzen, weitgehend unterdrückt.

In allen genannten Beispielen läßt sich das jeweilige Gleichgewicht in einfacher und effizienter Weise durch den Eingriff eines Inertgases zugunsten des gewünschten Ablaufs der Reaktion verschieben. Das Inertgas nimmt an der Reaktion nicht teil und läßt sich durch einfache Verfahrensschritte vollständig zurückgewinnen; es wird also nicht verbraucht, was auch den Einsatz teurer Inertgase ermöglicht. Der Verschiebung des jeweiligen Gleichgewichts werden keine physikalischen oder chemischen Kräfte entgegengesetzt. Die gewünschten Reaktionsprodukte werden in hoher Ausbeute und Reinheit gewonnen. Außerdem kann in katalysierten Systemen die Katalysatormenge drastisch, teilweise auf 1/10 der im Stand der Technik verwendeten Menge, reduziert werden, was nicht nur eine erhebliche Kosteneinsparung beim Katalysator-Verbrauch mit sich bringt, sondern auch die Probleme bei der Abtrennung das Katalysators erheblich verringert.

Die Erfindung wird anhand der nachfolgenden Beispiele erläutert, ohne auf diese beschränkt zu sein.

**Beispiel 1**

**Herstellung eines Caprylsäure-Caprinsäure-Triglycerids aus den entsprechenden Methylestern**

In einen Schleifenreaktor gemäß der beigefügten Figur 1 A mit einem Reaktorfüllvolumen von 65 1 und mit einem Gaskreislauf, in dem sich jeweils zuschaltbar Einheiten zur Entfernung von Sauerstoff, zur Trocknung, zum Abreagieren reaktiver Komponenten und zur Kondensation befanden und der im Flüssigkeitskreislauf eine gegebenenfalls zuschaltbare Filtrationseinheit 6 umfaßte, wurden 40 kg (231 Mol) eines Caprylsäuremethylester-Caprinsäuremethylester-Gemischs (Gewichtsteile $C_8 : C_{10} \approx 1 : 1$) und 6,07 kg (66 Mol) Glycerin eingefüllt. Der Reaktor wurde sodann mit $N_2$ gespült und gefüllt, und die Temperatur wurde danach auf 60 °C eingestellt.

Während eines 15 min dauernden Reinigungsschrittes wurde unter Umpumpen der Flüssigphase mit einer Umwälzleistung von ca. 3,6 $m^3$/h der Gaskreislauf betrieben und nacheinander zuerst die Trocknungseinheit und dann die Einheit zur Entfernung von Sauerstoff zugeschaltet. Die Strömungsgeschwindigkeit am Austrag des Strahlsaugers lag in der Größenordnung 17 m/s

Nach Beendigung des Reinigungsschrittes wurde dem Gaskreislauf die Kondensationseinheit zugeschaltet, während die Trocknungseinheit und die Einheit zur Entfernung von Sauerstoff nach der Vorbehandlung abgeschaltet wurden. Der Reaktionsmischung wurden 0,46 kg 50 %iges methanollösliches inertisiertes Kaliummethylat als Umesterungskatalysator zugesetzt, und die Temperatur wurde auf die Reaktionstemperatur von 100 °C angehoben.

Die Umesterung wurde für die Zeit von 30 min bei 100 °C durchgeführt. Während dieser Zeit wurde das sich bildende Methanol kontinuierlich in den Gaskreislauf ausgeschleust und in der Kondensationseinheit bei 0 °C kondensiert.

Sobald im Verlauf der Reaktion kein MeOH mehr ausgetragen wurde, wurden dem Reaktor 800 g kristalline Citronensäure zugeführt. Diese wurde nach 10 min Umwälzung in der Reaktionsmischung über die dem Flüssigkreis zuschaltbare Filtrationseinheit als K-Citrat entfernt.

Die Reaktionsmischung, bestehend aus dem gebildeten Triglycerid, überschüssigen Methylestern und niedermolekularen Verunreinigungen, nicht umgesetzten Edukten, und gebildeten Nebenprodukten, wurde auf 200 °C erwärmt. Durch Betreiben des Gaskreislaufs wurden - abgesehen von dem gewünschten Triglycerid - alle anderen genannten Komponenten, die ein niedrigeres Molekulargewicht als das Triglycerid hatten, in den Gaskreislauf ausgeschleust und durch Kondensation in der Kondensationseinheit vom Stickstoff einzeln abgetrennt.

Das nach Kühlung erhaltene Triglycerid (32 kg) war ausweislich einer dünnschichtchromatographischen Prüfung frei von Nebenprodukten, insbesondere von Methylester, Partialglyceriden und freien Fettsäuren.

**Vergleichsbeispiel 1 und Beispiel 2**

**Herstellung von Sorbitanestern unter reduzierenden Bedingungen**

In den in Beispiel 1 beschriebenen Schleifenreaktor wurden 17,5 kg Sorbit (70 % in $H_2O$) und 27,7 kg Ölsäure zusammen mit 0,3 kg NaOH (50 %ig in $H_2O$) und 0,13 kg kristalliner phosphoriger Säure gegeben. Nach Spülen und Inertisieren mit reinem Stickstoff wurde die Reaktion über die Zeit von 1 h bei 180 °C und dann über die Zeit von 2 h bei 240 °C durchgeführt. Dabei wurden kontinuierlich das Lösungswasser und das Reaktionswasser mit reinem Stickstoff in den Gaskreislauf ausgeschleust, dem zwei bei unterschiedlicher Temperatur betriebene Kondensatoren zugeschaltet worden waren. Im ersten Kondensator (110 °C) wurde die übergetriebene Fettsäure abgeschieden; im zweiten Kondensator (10 °C) wurde das Wasser abgeschieden. Die so zurückgewonnene Fettsäure wurde dem Reaktor 1 h vor Beendigung der Reaktion wieder zugeführt. Der erhaltene Sorbitanester hatte eine OHZ von 205 bis 208 und eine Farbe von 10 Hess-Ives.

Die vorstehend beschriebene Umsetzung wurde wiederholt, mit dem Unterschied, daß dem im Kreislauf geführten Stickstoff Wasserstoff (Partialdruck: 0,1 bar) zugesetzt wurde. Der erhaltene Sorbitanester hatte eine OHZ von 205 bis 208 und eine Farbe von 5 Hess-Ives.

**Beispiel 3 und Vergleichsbeispiel 2**

**Herstellung von Kokosfettsäuredimethylaminopropylamid (Entfernung von Sauerstoff)**

In den in Beispiel 1 angegebenen Schleifenreaktor wurden 33 kg Kokosfett und 18 kg Dimethylaminopropylamin gefüllt. Aus dem Gemisch wurde bei 90 °C über den Gaskreislauf mittels Stickstoff der Sauerstoff entfernt.

Die anschließende Umsetzung zu Kokosfettsäuredimethylaminopropylamid wurde bei 180 °C über die Zeit von 1 h ohne Nutzung der Kondensationseinheit durchgeführt. Das erhaltene rohe Amid hatte eine Farbzahl von deutlich unter 100 Hazen.

Die vorstehend beschriebene Umsetzung wurde wiederholt, mit der Abwandlung, daß der Sauerstoff aus der Eduktmischung nicht entfernt wurde. Das Produkt hatte eine Farbzahl von etwa 500 Hazen.

**Beispiel 4**

**Trocknung einer Reaktions-Eduktmischung**

In dem in Beispiel 1 angegebenen Schleifenreaktor wurden 40 kg $C_{12/14}$-Fettalkohol und 24 g KOH (50 %ig in $H_2O$) bei einer Temperatur von 140 °C unter $N_2$-Umlauf 30 min lang umgewälzt. Der Stickstoff wurde über die dem Gaskreislauf zugeschaltete Kondensationseinheit und anschließend über die in Reihe geschaltete Trocknungseinheit geführt. Der Wassergehalt vor der Behandlung war 0,1 %. Durch die Trocknung konnte der Wassergehalt auf < 0,001 % abgesenkt werden. Diese niedrigen Wassergehalte ermöglichten es, den Gehalt an Polyethylenglykol in mit dieser Eduktmischung hergestellten Ethoxylaten drastisch abzusenken.

**Beispiel 5**

**Herstellung eines Ethoxylats eines $C_{12/14}$-Fettalkohols mit 2 Mol Ethylenoxid**

In dem in Beispiel 1 angegebenen Schleifenreaktor wurden 40 kg Laurylalkohol/Myristylalkohol inertisiert. Danach wurden 24 g KOH (50-%ig in $H_2O$) zugegeben. Das Gemisch wurde durch Umwälzen unter Stickstoff wie in Beispiel 4 getrocknet. Danach wurde die Umsetzung unter kontinuierlicher Zufuhr von Ethylenoxid (insgesamt 8,8 kg) unter Einhaltung einer Reaktionstemperatur von 160 °C durchgeführt.

Die Reaktion wurde in der Weise beendet, daß man die Zufuhr von Ethylenoxid beendete, jedoch das im System noch vorhandene Ethylenoxid vollständig abreagieren ließ. Danach wurde der Katalysator durch Zugabe von Essigsäure zerstört.

Danach wurde die Temperatur der Reaktionsmischung auf 230°C angehoben. Bei dieser Temperatur wurde über einen Zeitraum von 1 h Fettalkohol und einfach ethoxylierter Fettalkohol mit dem umlaufenden Stickstoff unter gleichzeitiger Einleitung von Wasserdampf vor der Strahldüse aus dem Reaktionsgemisch ausgeschleust und unter Kondensation in der Kondensationseinheit vom Stickstoff abgetrennt.

Das Reaktionsgemisch hatte vor der Abtrennung der Edukte und Nebenprodukte einen Gehalt an $C_{12/14}$-Fettalkohol von 12 Gew.-% und einen Gehalt an einfach ethoxyliertem Addukt (ROH + 1 EO) von 10 Gew.-%. Demgegenüber war nach der Abtrennung der Edukte und Nebenprodukte der Gehalt an $C_{12/14}$-Fettalkohol auf 3 Gew.-% und der Gehalt an einfach ethoxyliertem Addukt (ROH + 1 EO) auf 7 Gew.-% reduziert. Diese Reduktion der lipophilen Bestandteile im Reaktionsprodukt zeigt sich in einer deutlich verbesserten Wasserlöslichkeit

**Patentansprüche**

1. Verfahren zur Durchführung einer durch ein Gleichgewicht gekennzeichneten physikalischen oder chemischen Reaktion, in einem als Schleifenreaktor ausgebildeten Reaktionssystem, umfassend einen Reaktorbehälter, mindestens eine mit dem Reaktorbehälter über je einen Auslaß und je einen Einlaß verbundene Schleife mit Einrichtungen zum Umpumpen des fluiden Reaktionsguts, mindestens einen Wärmetauscher, gegebenenfalls Einrichtungen zum Einspeisen des Reaktionsfluids in den Reaktorbehälter und einen getrennten Gaskreislauf, der mit dem über dem fluiden Reaktionsgemisch befindlichen Gasraum des Reaktorbehälters in Verbindung steht und getrennte Einrichtungen zum Einspeisen von Gas in den Gaskreislauf, zum Abziehen von Gas aus dem Gaskreislauf und zur Behandlung des im Gaskreislauf umlaufenden Gases aufweist, worin
ein Inertgas im Gaskreislauf geführt und behandelt wird, zum Eingreifen in die im Reaktorbehälter ablaufende, durch ein Gleichgewicht gekennzeichnete Reaktion in den Reaktorbehälter eingespeist wird und nach Eingriff in das Gleichgewicht der im Reaktorbehälter ablaufenden Gleichgewichtsreaktion aus dem Gasraum des Reaktorbehälters in den Gaskreislauf abgezogen wird, und worin
das Inertgas bei seinem Abziehen aus dem Reaktionsbehälter in den getrennten Gaskreislauf in der Weise in die durch ein Gleichgewicht gekennzeichnete Reaktion im Reaktorbehälter eingreift, daß es die Reaktionsmischung von darin gelöstem Gas und/oder von darin transportierten flüchtigen Stoffen befreit, indem es dieses/diese aus der Reaktionsmischung austrägt.

2. Verfahren nach Patentanspruch 1, worin das Inertgas turbulent in die Reaktionsmischung eingetragen wird.

3. Verfahren nach Patentanspruch 1 oder 2, worin das Inertgas die im Reaktorbehälter befindliche Reaktionsmischung von Sauerstoff befreit und/oder trocknet.

4. Verfahren nach Patentanspruch 1 oder 2, worin das Inertgas überschüssiges Reaktionsgas aus der im Reaktorbehälter befindlichen Reaktionsmischung austrägt.

5. Verfahren nach Patentanspruch 1 oder 2, worin das Inertgas zusammen mit flüchtigen Stoffen aus dem Gasraum über der Reaktionsmischung abgezogen und vor Wiedereinspeisen in den Reaktionsbehälter von mitgerissenen flüchtigen Stoffen befreit wird und in der Weise in die durch ein Gleichgewicht gekennzeichnete Reaktion im Reaktolbehälter eingreift, daß es der Reaktionsmischung einen flüchtigen Reaktionspartner entzieht.

6. Verfahren nach Patentanspruch 5, worin der Entzug des flüchtigen Reaktionspartners mit dem Inertgas während des Verlaufs der Reaktion oder nach vollständigem Ablauf der Reaktion erfolgt und der durch das Inertgas der Reaktionsmischung entzogene und im Gaskreislauf abgetrennte Reaktionspartner ein im Überschuß eingesetztes Edukt der durch ein Gleichgewicht gekennzeichneten Reaktion ist.

**7.** Verfahren nach Patentanspruch 5, worin der Entzug des flüchtigen Reaktionspartners mit dem Inertgas während des Verlaufs der Reaktion und/oder nach vollständigem Ablauf der Reaktion erfolgt und der durch das Inertgas der Reaktionsmischung entzogene und im Gaskreislauf abgetrennte Reaktionspartner ein im Verlauf der Reaktion entstandenes Produkt und/oder Nebenprodukt der durch ein Gleichgewicht gekennzeichneten Reaktion ist.

**8.** Verfahren nach einem der Patentansprüche 1 bis 7, worin die Behandlung des Inertgases im Gaskreislauf neben der Befreiung des Inertgases von darin enthaltenen Komponenten aus dem Reaktionsgemisch zusätzlich in einer nachfolgenden Beaufschlagung des Inertgases mit flüchtigen Stoffen für das Reaktionsgemisch besteht.

**9.** Verfahren nach einem oder mehreren der Patentansprüche 1 bis 8, worin die Befreiung des Inertgases von darin enthaltenen Komponenten aus dem Reaktionsgemisch im Gaskreislauf durch Kondensation, Ausfrieren, Adsorption, Trocknung, reaktives Abreagieren und/oder Molekularsieb-Behandlung erfolgt.

**10.** Verfahren nach Patentanspruch 1, worin der Reaktionsmischung der flüchtige Reaktionspartner unter Verbesserung des Kontakts zwischen gasförmigen und bereits gelösten Reaktionspartnern in der Reaktionsmischung zugeführt wird.

**11.** Verfahren nach Patentanspruch 10, worin der durch das Inertgas zugeführte gasförmige Reaktionspartner in gesteuert niedrigem Partialdruck vorliegt.

**12.** Verfahren nach Patentanspruch 10, worin der durch das Inertgas zugeführte gasförmige Reaktionspartner in durch das Auslaufen der Reaktion bedingtem niedrigem Partialdruck vorliegt.

**13.** Verfahren nach einem oder mehreren der Patentansprüche 1 bis 12, worin die Behandlung des Inertgases unter Erhitzen des Inertgases oder unter Abkühlung des Inertgases erfolgt.

**14.** Verfahren nach einem oder mehreren der Patentansprüche 1 bis 13, worin das Inertgas Stickstoff ($N_2$), Argon (Ar), $CO_2$, $H_2O$ und ein sonstiger inerter, bei den Reaktionsbedingungen gasförmiger Stoff oder eine Mischung eines oder mehrerer Inertgase, bevorzugt jedoch Stickstoff ist.

**Claims**

**1.** A process for conducting a physical or chemical reaction **characterized by** an equilibrium in a reaction system designed as a loop reactor, said loop reactor comprising a reactor vessel, at least one loop connected to said reactor vessel each by means of an outlet and an inlet, said loop comprising means for pumping over a fluid reaction material, at least one heat exchanger, optionally means for feeding said reaction fluid into the reactor vessel and a separate gas loop which is connected to the gas space of the reactor vessel above the fluid reaction mixture and has separate means for feeding a gas into the gas loop, for withdrawing gas from the gas loop and for treating said gas circulating in the gas loop, wherein an inert gas is circulated and treated in said gas loop, is fed into the reactor vessel for influencing said reaction conducted in said reactor vessel and being **characterized by** an equilibrium and, after influencing the equilibrium of said equilibrium reaction conducted in said reactor vessel, is withdrawn from the gas space of said reactor vessel into the gas loop, and wherein the inert gas, when withdrawn from the reactor vessel into the separate gas loop, influences the reaction **characterized by** an equilibrium and conducted in the reactor vessel in such a manner that the reaction mixture is freed from gas dissolved therein and/ or from volatile substances transported therein by carrying it/them off the reaction mixture.

**2.** The process according to claim 1, wherein the inert gas is charged into the reaction mixture in a turbulent manner.

**3.** The process according to claim 1 or claim 2, wherein the inert gas makes the reaction mixture contained in the reactor vessel free of oxygen and/or dries the reaction mixture.

**4.** The process according to claim 1 or claim 2, wherein the inert gas carries excessive reaction gas off the reaction mixture contained in the reactor vessel.

**5.** The process according to claim 1 or claim 2, wherein the inert gas, together with volatile substances, is withdrawn from the gas space above the reaction mixture and, before being re-fed into the reaction vessel, is freed from entrained volatile substances and influences the reaction **characterized by** an equilibrium and conducted in said reactor vessel in such a manner that it removes a volatile reactant from the reaction mixture.

6. The process according to claim 5, wherein the removal of said volatile reactant together with the inert gas is effected in the course of said reaction or after a completion of said reaction, and the reactant removed from the reaction mixture by the inert gas and separated in said gas loop is an educt for the reaction **characterized by** an equilibrium which was supplied in excess.

7. The process according to claim 5, wherein the removal of said volatile reactant together with the inert gas is conducted in the course of said reaction and/or after a completion of said reaction, and wherein the reactant removed from the reaction mixture by the inert gas and separated in said gas loop is a product and/or by-product of the reaction **characterized by** an equilibrium, said product/by-product being generated in the course of said reaction.

8. The process of any of claims 1 to 7, wherein the treatment of the inert gas in the gas loop, beside the step of freeing the inert gas from components of the reaction mixture contained therein, consists of an additional step of subsequently adding volatile substances for the reaction mixture to the inert gas.

9. The process of one or more of claims 1 to 8, wherein the step of making, in said gas loop, the inert gas free of components of the reaction mixture contained therein is carried out by condensation, by freezing-out, by adsorption, by drying, by actively reacting and/or by treatment with a molecular sieve.

10. The process of claim 1, wherein said volatile reactant is supplied to the reaction mixture with improving the contact between gaseous reactants and reactants already dissolved in the reaction mixture.

11. The process according to claim 10, wherein the gaseous reactant supplied by the inert gas is present at a partial pressure which is controlled to be low.

12. The process according to claim 10, wherein the gaseous reactant supplied by the inert gas is present at a low partial pressure conditional on a ceasing of the reaction.

13. The process according to any of the claims 1 to 12, wherein the treatment of said inert gas is conducted by heating the inert gas or cooling the inert gas.

14. The process according to any one or more of the claims 1 to 13, wherein the inert gas is nitrogen ($N_2$), argon (Ar), $CO_2$, $H_2O$ and another inert substance gaseous at the reaction conditions or is a mixture of one or more inert gases, preferably wherein the inert gas is nitrogen.

**Revendications**

1. Procédé pour réaliser une réaction physique ou chimique équilibrée dans un système réactionnel conçu comme un réacteur à boucle, comprenant une cuve de réacteur, au moins une boucle, reliant entrée et sortie de la cuve de réacteur, munie de dispositifs pour transvaser par pompage le milieu réactionnel fluide, au moins un échangeur de chaleur, le cas échéant des dispositifs pour alimenter en fluide réactionnel la cuve de réacteur et un circuit de gaz distinct, qui est en relation avec l'espace gazeux se trouvant au-dessus du mélange réactionnel fluide dans la cuve de réacteur, et des dispositifs distincts pour introduire du gaz dans le circuit de gaz, retirer du gaz du circuit de gaz et traiter le gaz circulant dans le circuit de gaz, procédé dans lequel:

   un gaz inerte amené et traité dans le circuit de gaz, de façon à intervenir, dans une réaction équilibrée se déroulant dans la cuve de réacteur, est introduit dans la cuve de réacteur, puis, après intervention dans l'équilibre de la réaction équilibrée se déroulant dans la cuve de réacteur, est retiré de l'espace gazeux de la cuve de réacteur pour passer dans le circuit de gaz, et dans lequel
   le gaz inerte, lors de son retrait de la cuve de réaction pour passer dans le circuit de gaz distinct, intervient dans la réaction équilibrée se déroulant dans la cuve de réacteur de telle sorte qu'il débarrasse le mélange réactionnel du gaz qui y est dissous et/ou des matières volatiles qui y sont transportées, retirant ainsi ce gaz ou ces matières volatiles du mélange réactionnel.

2. Procédé selon la revendication 1, dans lequel le gaz inerte est introduit de façon turbulente dans le mélange réactionnel.

3.  Procédé selon la revendication 1 ou 2, dans lequel le gaz inerte débarrasse de l'oxygène et/ou sèche le mélange réactionnel se trouvant dans la cuve de réacteur.

4.  Procédé selon la revendication 1 ou 2, dans lequel le gaz inerte entraîne le gaz réactionnel en excès présent dans le mélange réactionnel se trouvant dans la cuve de réacteur.

5.  Procédé selon la revendication 1 ou 2, dans lequel le gaz inerte ainsi que les matières volatiles sont retirés de l'espace gazeux au-dessus du mélange réactionnel, et, avant sa réintroduction dans la cuve de réacteur, le gaz inerte est débarrassé des matières volatiles entraînées et intervient, dans la cuve de réacteur, dans la réaction équilibrée de façon à extraire du mélange réactionnel un partenaire réactionnel volatil.

6.  Procédé selon la revendication 5, dans lequel l'extraction du partenaire réactionnel volatil par le gaz inerte se produit au cours de la réaction ou après déroulement complet de la réaction et dans lequel le partenaire réactionnel extrait du mélange réactionnel par le gaz inerte et séparé dans le circuit de gaz est un agent d'extraction utilisé en excès dans la réaction équilibrée.

7.  Procédé selon la revendication 5, dans lequel le retrait du partenaire réactionnel volatil par le gaz inerte se produit au cours de la réaction et/ou après un déroulement complet de la réaction et le partenaire réactionnel retiré du mélange réactionnel par le gaz inerte et séparé dans le circuit de gaz est un produit formé au cours de la réaction et/ou un produit secondaire résultant de la réaction équilibrée.

8.  Procédé selon l'une des revendications 1 à 7, dans lequel le traitement du gaz inerte dans le circuit de gaz, outre la libération du gaz inerte des composés du mélange réactionnel qui y sont contenus, consiste en une alimentation ultérieure du gaz inerte en matières volatiles pour le mélange réactionnel.

9.  Procédé selon une ou plusieurs des revendications 1 à 8, dans lequel la libération du gaz inerte des composés du mélange réactionnel qui y sont contenus s'effectue dans le circuit de gaz par condensation, congélation, adsorption, séchage, libération réactive et/ou traitement sur tamis moléculaire.

10. Procédé selon la revendication 1, dans lequel le partenaire réactionnel volatil est ajouté au mélange réactionnel pour améliorer le contact entre des partenaires réactionnels gazeux et déjà dissous dans le mélange réactionnel.

11. Procédé selon la revendication 10, dans lequel le partenaire réactionnel gazeux introduit avec le gaz inerte se présente sous une faible pression partielle contrôlée.

12. Procédé selon la revendication 10, dans lequel le partenaire réactionnel gazeux introduit avec le gaz inerte se présente sous une faible pression partielle conditionnée par le déroulement de la réaction.

13. Procédé selon une ou plusieurs des revendications 1 à 12, dans lequel le traitement du gaz inerte s'effectue par chauffage du gaz inerte ou par refroidissement du gaz inerte.

14. Procédé selon une ou plusieurs des revendications 1 à 13, dans lequel le gaz inerte consiste en azote ($N_2$), argon (Ar), $CO_2$, $H_2O$ ou autre matière inerte gazeuse dans les conditions réactionnelles ou un mélange d'un ou plusieurs gaz inertes, mais consiste de préférence en azote.

FIGUR 1

FIGUR 2

## FIGUR 3

A — Trockeneinheit

B — Einheit z. Entfernen von $O_2$

C — Kondensator-Einheit I

D — Kondensator-Einheit II